# EUROPÄISCHE PATENTSCHRIFT

(11) **EP 4 127 311 B1**
(45) Veröffentlichungstag und Bekanntmachung des Hinweises auf die Patenterteilung: **25.11.2026**
(21) Anmeldenummer: 21707214.9
(22) Anmeldetag: 19.02.2021
(51) Int. Cl.: D21G 9/00, G01N 33/34

(54) **VERFAHREN UND VORRICHTUNG ZUR ERMITTLUNG DER FASERORIENTIERUNG**
METHOD AND APPARATUS FOR DETERMINING THE FIBER ORIENTATION
PROCÉDÉ ET APPAREIL DE DÉTERMINATION DE L'ORIENTATION DE FIBRES

(30) Priorität: 23.03.2020 DE 102020107855
(43) Veröffentlichungstag der Anmeldung: 08.02.2023
(73) Patentinhaber: Voith Patent GmbH, 89522 Heidenheim (DE)
(72) Erfinder: AJIT, Sime, 89522 Heidenheim (DE); SOLDAN, Samuel, 89537 Giengen (DE); ABELE, Michael, 89522 Heidenheim (DE); KENDEL, Friedrich, 89077 Ulm (DE)
(74) Vertreter: Voith Patent GmbH - Patentabteilung
(86) Internationale Anmeldenummer: PCT/EP2021/054104
(87) Internationale Veröffentlichungsnummer: WO 2021/190832

(56) Entgegenhaltungen:
- AT-A1- 507 018
- US-A- 4 955 720
- US-B2- 7 695 592

## Beschreibung

Die Erfindung betrifft ein Verfahren zur Ermittlung der Faserorientierung einer bewegten Faserstoffbahn gemäß dem Oberbegriff des Anspruchs 1 sowie eine zugehörige Vorrichtung gemäß dem Oberbegriff des Anspruchs 7.

Die Faserorientierung ist eine wichtige Qualitätsgröße bei der Beurteilung von Faserstoffbahnen, insbesondere bei Papierbahnen. Bei einer Papierbahn sind die einzelnen Zellstofffasern aufgrund des Herstellungsprozesses zwar grob in Maschinenlaufrichtung ausgerichtet. In Abhängigkeit von verschiedenen Prozesseinstellungen oder Maschinenkonfigurationen kann diese Orientierung aber auch merklich von der Maschinenlaufrichtung abweichen.

Die Orientierung der Fasern kann viele Eigenschaften des Papiers beeinflussen. Speziell die Dimensionsstabilität kann sich durch eine mangelhafte Verteilung der Faserorientierung verschlechtern. Dies ist unter anderem in der Tatsache begründet, dass Fasern bei Erhitzung in ihrem Durchmesser relative stark schrumpfen im Vergleich zu ihrer Länge. Wenn beispielsweise bei einem Kopierpapier auf der Oberseite die Fasern sehr stark in Maschinenrichtung orientiert sind, wie das Blatt beim Kopieren an dieser Oberseite stark in der Breite schrumpfen. Sind nun die Fasern an der Unterseite des Blattes weniger stark oder in eine andere Richtung orientiert, so schrumpft das Blatt an der Unterseite weniger stark in Breitenrichtung. Dadurch entstehen Spannungen im Blatt, die dann in Form des sogenannten 'Curls', also einer Wellen- oder Rollenbildung des Blattes sichtbar werden. Speziell beim Doppelseitigen Kopieren kann es hierbei auch zu Papierstau im Kopierer führen. Neben dem hier beschriebenen Beispiel ist die Faserorientierung bei nahezu allen Papier- Verpackungs- und Kartonsorten ein wichtiges Merkmal. Daher ist es für den Hersteller wünschenswert, die Faserorientierung bereits während des Herstellungsprozesses der Faserstoffbahn zu kennen, um gegebenenfalls gegensteuern zu können.

Aus dem Stand der Technik sind hierzu einige Ansätze bekannt. Insbesondere ist hier die Publikation US 7,695,592 zu nennen. Hier wird ein optisches System beschrieben, bei dem die Oberfläche der laufenden Papierbahn beleuchtet und mit einer Kamera fotografiert wird. Aus dem digitalen Bild kann dann mittels geeigneter mathematischer Methoden ein Wert für die Faserorientierung berechnet werden. Das da beschriebene Verfahren bietet den Vorteil, dass es einen vergleichsweise geringen Aufwand erfordert. In den letzten 15 Jahren seit der Anmeldung der obigen Patentschrift hat sich die Produktionsgeschwindigkeit von Papiermaschinen jedoch stark erhöht. Bei hohen Geschwindigkeiten kommt es, wie die US 7,695,592 selbst schreibt, zu verschwommenen Aufnahmen ('blurring') der Bahnoberfläche. Dies beeinflusst das Messergebnis stark, und kann auch durch mathematisches Preprozessing der Bilder nicht vollständig korrigiert werden.

Es ist daher Aufgabe der vorliegenden Erfindung ein Verfahren und eine Vorrichtung vorzuschlagen, um die Probleme des Standes der Technik zu beheben. Insbesondere ist es Aufgabe der Erfindung, eine einfache Messung der Faserorientierung auch bei hohen Produktionsgeschwindigkeiten zu ermöglichen.

Die Aufgabe wird erfindungsgemäß durch ein Verfahren entsprechend Anspruch 1 sowie eine Vorrichtung entsprechend Anspruch 8 gelöst.

Weitere vorteilhafte Merkmale der erfindungsgemäßen Ausführung finden sich in den Unteransprüchen.

Hinsichtlich des Verfahrens wird die Aufgabe gelöst durch ein Verfahren zur Ermittlung der Faserorientierung einer bewegten Faserstoffbahn, insbesondere einer Papier-, Karton- oder Zellstoffbahn. Das Verfahren umfasst dabei die Schritte
- Beleuchten einer Fläche A der Faserstoffbahn für einen Zeitraum Δt
- Aufnahme eines digitalen Bildes der beleuchteten Fläche A

Erfindungsgemäß ist dabei vorgesehen, dass der Zeitraum Δt maximal 1 [µs], insbesondere maximal 500 [ns] beträgt, und das digitale Bild eine so hohe Auflösung aufweist, dass die Kantenlänge eines Pixels wenige als 20 [µm], insbesondere weniger als 10[µm] entspricht.

In vorteilhaften Ausführungen kann das Berechnen einer Faserorientierung aus den Daten des digitalen Bildes vorgesehen sein.

Die Berechnung einer Faserorientierung aus den Daten eines digitalen Bildes ist an sich bekannt. Hierzu sei beispielsweise auf den Übersichtsartikel *"*Kellomäki, M., Kärkkäinen, S., Penttinen, A., & Lappalainen, T. (2003). Determination of fiber orientation distribution from images of fiber networks. Jyväskylä: University of Jyväskylä. Publications of Laboratory of Data Analysis, Vol. 4" verwiesen.

Auch in der US 7,695,592 ist ein geeignetes Verfahren beschrieben. Die Faserorientierung bei der Papierherstellung bezieht sich dabei auf die bevorzugte Orientierung der einzelnen Fasern auf der Bahn. Aufgrund der Strömungsmuster im Stoffauflauf und des auf das Sieb auftreffenden Strahls neigen die Fasern dazu, sich in Maschinenrichtung (MD) gegenüber anderen Richtungen in der Bahn auszurichten. Wenn alle Fasern in der Bahn perfekt verteilt wären, hätte der Papierbogen in alle Richtungen die gleichen Eigenschaften. Dies wird als isotropes Blatt bezeichnet, und seine Faserverteilung kann in Form eines Kreises in einem Polardiagramm aufgezeichnet werden. Ein Faserverhältnis, das das Verhältnis der maximalen zur minimalen Faserverteilung im Abstand von 90° ist, kann für ein Papierblatt definiert werden. Ein isotropes Blatt hat ein Faserverhältnis von eins.

Wenn in einer Richtung mehr Fasern als in anderen Richtungen vorhanden sind, sind die Fasern ungleichmäßig verteilt und die Folie ist anisotrop. Eine solche anisotrope Faserverteilung kann in einem Polardiagramm als Ellipse aufgetragen werden.

Aus der Geometrie der Ellipse können dann relevante Kenngrößen der Faserorientierung ermittelt werden. Der Winkel θ, den die Hauptachse der Ellipse mit der Maschinenrichtung beschreibt, gibt den -mittleren- Winkel der Faserorientierung an. Das Längenverhältnis der Hauptachse zur Nebenachse beschreibt den Grad der Anisotropie, bzw. den Grad der Orientierung. Eine hohe Anisotropie zeigt an, dass ein großer Anteil der Fasern auch tatsächlich in Richtung des Winkels θ ausgerichtet ist. Eine Anisotropie nahe 1 (d.h. eine Ellipse, die nahezu kreisförmig ist) gibt an, dass die Fasern kaum eine Vorzugsrichtung aufweisen. Das Blatt ist dann nahezu isotrop. Das hier beschriebene Verfahren soll nur beispielhaft aufzeigen, wie eine Berechnung der Faserorientierung aus einem digitalen Bild erfolgen kann. Alternativ sind aber auch andere Auswerteverfahren, wie zum Beispiel Gradienten-basierte Verfahren geeignet. Die Erfindung ist dabei nicht auf eine spezifische Berechnungsmethode beschränkt.

Prinzipiell kann mit den bekannten Verfahren aus jedem Bild der Papieroberfläche eine Faserorientierung - also z.B. ein Winkel und eine Anisotropie -berechnet werden. Wird jedoch die Oberfläche der Bahn durch das Bild nicht exakt genug abgebildet, so hat die aus diesem Bild ermittelte Faserorientierung nicht viel mit der realen Orientierung der Fasern zu tun. Wird beispielsweise eine zu lange Belichtungszeit gewählt, so bewegt sich die Bahn während der Belichtung in MD Richtung unter der Kamera hinweg. Ein punktförmiges Objekt auf der Bahn wird dabei als MD-orientierter Streifen auf dem digitalen Bild abgebildet. Durch diesen Effekt bzw. Defekt der Bildaufnahme ist die Struktur auf dem digitalen Bild viel stärker in MD orientiert, als die eigentliche Faserstoffbahn. Die daraus berechnete Faserorientierung weicht dadurch deutlich von der realen Faserorientierung ab. Für eine zuverlässige Ermittlung der Faserorientierung ist daher weniger ein ausgefeiltes Berechnungsverfahren notwendig, als vielmehr eine möglichst genaue Wiedergabe der Oberfläche der Faserstoffbahn auf dem digitalen Bild.

Zum Erzielen einer solchen genauen Wiedergabe sind verschiedene Aspekte zu beachten.

Zum einen muss die Auflösung des Bildes hoch genug sein, und zwar deutlich höher, als bisher im Stand der Technik angenommen. Ging man bisher davon aus, dass die Auflösung so gewählt werden muss, dass ein Pixel in etwa dem Durchmesser einer Faser von ca. 40µm entsprechen muss haben die Erfinder erkannt, dass für eine zuverlässige Bestimmung der Faserorientierung die Kantenlänge eines Pixels unterhalb von 20µm, vorteilhafterweise sogar unterhalb von 10 µm liegt. In besonders bevorzugten Ausführungen kann die Kantenlänge eines Pixels auch unter 8µm betragen.

Zudem muss diese hohe Auflösung kombiniert werden mit einem Bild, das nicht verschwommen ist.

Die Größenordnung soll dabei die folgende Beispielrechnung verdeutlichen.

Bei einer Maschinengeschwindigkeit von 1000 m/min, die heutzutage eher unterdurchschnittlich ist, bewegt sich die Bahn ca. 16 m/s, oder 16 µm/µs. Somit bewegt sich die Bahn in einer µs knapp um einen halben Faserdurchmesser. Bei der erfindungsgemäßen Auflösung des Bildes entspricht dies in etwa auch der Dimension eines Pixels.

Daher sind bei der Aufnahme extrem kurze Belichtungszeiten notwendig. Die Erfinder haben dabei herausgefunden, dass es nicht, oder nicht mit realisierbarem Aufwand möglich ist, diese kurzen Belichtungszeiten allein über entsprechend kurze Verschlusszeiten einer Kamera zu realisieren. Daher sieht das vorgeschlagene Verfahren vor, dass die Fläche A, von der ein digitales Bild aufgenommen wird für einen Zeitraum Δt von maximal 1 [µs], insbesondere von 500 [ns] oder weniger beleuchtet wird. Mit der aktuellen Technologie sind sogar deutlich kürzere Beleuchtungszeiten von 200 [ns] oder weniger mit vertretbarem Aufwand realisierbar.

Mit einem derartigen Verfahren sind zuverlässige Messungen der Faserorientierung auch für Maschinengeschwindigkeit bzw. Bahngeschwindigkeiten von über 1200 m/min oder auch mehr als 1400 m/min möglich.

Als vorteilhaft hat es sich herausgestellt, dass für die Beleuchtung Licht mit Wellenlängen im Bereich zwischen 450 [nm] und 780 [nm], insbesondere zwischen 450 [nm] und 550 [nm] oder zwischen 600 [nm] und 700 [nm] verwendet wird. Dies hat einerseits den Vorteil, dass übliche CMOS Chips von Digitalkameras für Licht in diesem Spektralbereich sehr empfindlich sind, und so eine ausreichende Lichtmenge erfasst werden kann.

Zudem wird etlichen Papiersorten ein sogenannter 'Optischer Aufheller' zugemischt. Dies sind meist fluoreszierende Substanzen, deren Funktion die Steigerung des Weißgrads, insbesondere durch Kompensation des Gelbstichs, von Materialien ist. Die hierfür am meisten verwendeten Fluoreszenzstoffe emittieren in einem Bereich unter 480 [nm], speziell unter 450 [nm]. Um einer Störung des Messergebnisses durch diese Fluoreszenzeffekte zu vermeiden, ist die Beleuchtung mit Licht von 450 [nm], insbesondere > 500 [nm] Wellenlänge vorteilhaft.

Weiterhin kann bevorzugt eine schmalbandige Beleuchtung eingesetzt werden, bei der die zur Beleuchtung verwendeten Wellenlängen eine Bandbreite von maximal 100 [nm], insbesondere maximal 50 [nm] aufweisen.

Weiterhin ist es vorteilhaft, wenn das Licht der Beleuchtung unter einem Winkel α auf die Faserstoffbahn trifft, welcher maximal 45°, insbesondere zwischen 25° und 40° von der Senkrechten abweicht.

Bei einem flacheren Lichteinfall mit einem Winkel α von mehr als 45° zur Senkrechten kommt es verstärkt zu einem Schattenwurf der Papieroberfläche - z.B. durch aus dem Blatt herausragende Fasern bzw. Faserteile. Dieser Schattenwurf kann sich störend auf die Berechnung der Faserorientierung aus dem digitalen Bild auswirken, bzw. der berechnete Wert kann dadurch merklich von der tatsächlichen Faserorientierung abweichen.

Weiterhin ist ein solcher steiler Lichteinfall auch dann vorteilhaft, wenn sich zwischen den Lichtquellen und der Faserstoffbahn noch Einbauten befinden, wie z.B. eine Glasplatte zum Schutz der Optischen Geräte.

Theoretisch optimal wäre sicherlich ein völlig senkrechter Einfall des Lichts auf die Faserstoffbahn. Dies ist aber einerseits konstruktiv schwierig zu realisieren. Andererseits würde bei einer solchen Anordnung reflektiertes Licht direkt in die Kamera reflektiert, was die Messung stören könnte.

Der bevorzugte Bereich zwischen 45° und 25° stellt in gewissem Sinne einen optimalen Kompromiss dar.

Um zuverlässige Messwerte zu erhalten ist es vorteilhaft, wenn die beleuchtete Fläche A ein Quadrat von 5mmx5mm, insbesondere von 10mmx10mm umfasst.

In bevorzugten Anwendungen wurde eine Bildgröße des digitalen Bildes von 11mm x 9 mm verwendet, die beleuchtete Fläche A war hier zumindest ebenso groß.

Eine größere Fläche ist prinzipiell vorteilhaft da damit eine ausreichende Zahl von Fasern abgebildet werden kann. Die liefert insbesondere bei der Berechnung der Faserorientierung genauere Werte.

Hinsichtlich der Vorrichtung wird die Aufgabe gelöst durch eine Vorrichtung zur Ermittlung der Faserorientierung eine bewegten Faserstoffbahn, insbesondere einer Papier-, Karton- oder Zellstoffbahn, umfassend Beleuchtungsmittel zum Beleuchten einer Fläche A der Faserstoffbahn, sowie eine Kamera zur Aufnahme eines digitalen Bildes der beleuchteten Fläche A der Faserstoffbahn. Erfindungsgemäß ist vorgesehen, dass die Beleuchtungsmittel dazu eingerichtet sind, die Faserstoffbahn für einen Zeitraum von 1 [µs] oder weniger zu beleuchten, sowie dass die Kamera derart eingerichtet ist, dass die Kantenlänge eines Pixels maximal 20 [µm], insbesondere maximal 10 [µm] entspricht, wobei die Vorrichtung zudem eine Recheneinheit umfasst, die dazu eingerichtet ist, aus den Daten eines digitalen Bildes der Kamera eine Faserorientierung zu berechnen.

Bzgl. der Berechnung der Faserorientierung aus dem digitalen Bild sei aus die entsprechenden Ausführungen der Verfahrensbeschreibung verwiesen.

Der Begriff "die Beleuchtungsmittel" beschreibt die eine Vorrichtung, welche eine oder mehrere Lichtquellen umfasst, zusammen mit Haltevorrichtungen sowie die zugehörigen Leitungen und elektrischen bzw. elektronischen Bauteile.

Häufig sind einige oder alle Elemente solcher Beleuchtungsmittel auf Leiterplatten (PCB- Printed Circuit Boards) angeordnet.

Die Kamera und die Beleuchtungsmittel sind üblicherweise zusammen in einer gemeinsamen Sensoreinheit verbaut, die in der Maschine in unmittelbarer Nähe zur Faserstoffbahn installiert werden kann. Diese Sensoreinheit kann traversierende ausgeführt sein, also beweglich entlang der Maschinenquerrichtung. Die Recheneinheit kann entweder ebenfalls in der Sensoreinheit verbaut sein, oder aber Außerhalb der Maschine vorgesehen sein. Im ersten Fall erfolgt die Berechnung der Faserorientierung in der Maschine selbst. Das hat den Vorteil, dass nur diese Faserorientierungswerte, und somit sehr geringe Datenmengen aus der Maschine übertragen werden müssen. Diese geringen Datenmengen können mit nahezu jeder Übertragungstechnik - kabelgebunden oder kabellos- aus der Maschine übertragen werden.

Im zweiten Fall müssen die gesamten Bilddaten vorzugsweise in Echtzeit aus der Sensoreinheit zur Recheneinheit übertragen werden. Dies stellt ungleich größere Anforderungen an die Datenübertragung.

In einer bevorzugten Ausführung kann vorgesehen sein, dass die Beleuchtungsmittel mehrere Lichtquellen, insbesondere mehrere LEDs umfassen, welche gleichmäßig und ringförmig um die Kamera herum angeordnet sind.

Ganz besonders vorteilhaft ist es hierbei, wenn es sich bei den verwendeten Lichtquellen um baugleiche Lichtquellen, speziell baugleiche LEDs handelt.

Der Vorteil der ringförmigen Anordnung ist, dass dadurch eine möglichste homogene, gleichmäßige Beleuchtung der Fläche A erzielt werden kann. Dadurch können in der Berechnung der Faserorientieren Artefakte vermieden werden, die z.B. durch eine einseitige Beleuchtung entstehen könnten.

Zum selben Zweck der Homogenisierung der Beleuchtung dient auch die gleichmäßige Verteilung der Lichtquellen entlang des Umfangs des Rings. Kommen beispielsweise 9 Lichtquellen in dem Beleuchtungsmittel zum Einsatz, so sollten diese vorteilhafterweise in einem Winkelabstand von 360°/9= 40° entlang des Kreisrings angeordnet sein.

Wie schon beschrieben, sollten die Lichtquellen vorteilhafterweise so angeordnet sein, dass ihr Licht unter einem Winkel α auf die Faserstoffbahn trifft, welcher maximal 45°, insbesondere zwischen 25° und 40° von der Senkrechten abweicht.

Da wie beschrieben eine möglichst homogene Ausleuchtung der Fläche A wünschenswert ist, sollte insbesondere alle Lichtquellen in demselben Winkel auf die Faserstoffbahn aufgerichtet sein.

Die Erfinder haben überraschenderweise herausgefunden, dass es vorteilhaft ist, wenn die Beleuchtungsmittel eine ungerade Anzahl von Lichtquellen aufweist. Im Hinblick auf die beschriebenen Vorteile der homogenen Ausleuchtung könnte man vermuten, dass eine gerade Anzahl von Lichtquellen vorteilhaft wäre. Jedoch hat sich gezeigt, dass dabei nachteilige Quantisierungseffekte auftreten. Das Vermeiden von gerichteter Quantisierung durch eine ungerade Anzahl von Lichtquellen hat sich als vorteilhaft erwiesen.

Die Anzahl der Lichtquellen hängt neben anderem von der Baugröße der Lichtquellen und dem vorhandenen Bauraum ab. Es können beispielsweise 11, 13, 15 oder 17 Lichtquellen eingesetzt werden.

In bevorzugten Ausführungen kann vorgesehen sein, dass zwischen den Beleuchtungsmitteln und der Faserstoffbahn und/oder zwischen der Kamera und der Faserstoffbahn eine Platte angeordnet ist, die für das Licht ganz oder weitgehend transparent ist.

Von einer bewegten Faserstoffbahn können sich - insbesondere bei hohen Geschwindigkeiten - Faserfragmente oder Fein- bzw. Füllstoffteilchen lösen, und sich auf den Maschinenteilen und Einbauten ablagern. Eine Platte wie oben beschrieben, die beispielsweise aus Glas oder aus Kunststoff ausgeführt sein kann, kann dazu diesen, Verschmutzung der Kamera und der Beleuchtungsmittel zu vermeiden. Gegebenenfalls können auch noch Reinigungsvorrichtungen vorgesehen sein, z.B. Düsen zur Spülung mit Luft oder Wasser, um Verschmutzungen der Platte zu entfernen.

Beim Betrieb der Messvorrichtung fließen häufig vergleichsweise große Ströme um die benötigte Lichtstärke und die kurzen Beleuchtungszeiten zu realisieren. Daher ist es vorteilhaft, wenn die Beleuchtungsmittel zumindest kurzzeitig dazu geeignet sind, mit einer Stromstärke von mindestens 10 [A] betrieben zu werden. Dies kann unter Einfluss auf die Dimensionierung der Leiterbahnen haben. Es kann zudem hilfreich sein, Mittel zum Abführen der entstehenden Wärme an oder in den Beleuchtungsmitteln vorzusehen.

Vorteilhafterweise kann vorgesehen sein, dass die optischen Eigenschaften und die Blenden-Einstellungen mit Blenden zwischen F4 und F16, der Linse der Kamera eine Auflösung ermöglicht, die einer Kantenlänge eines Pixels von maximal 20 [µm], insbesondere maximal 10 [µm] entspricht.

Über eine etwas geschlossene Blende kann dann trotz der Lichtverluste noch genug Tiefenschärfe erhalten werden um ein scharfes Bild aufzulösen. Dies ist vorteilhaft, da Zellstofffasern üblicherweise zwischen 10µm und 50µm dick sind, und teilweise aus der Oberfläche z.B. einer Papierbahn herausragen können. Somit weist eine solche Bahn im µm Bereich eine sehr grobe Topographie aufweist. Vorteilhafterweise ist die Kamera in der Lage, diese Topographie noch scharf darzustellen.

Weiterhin vorteilhaft ist die Vorrichtung, die eine Führungsschiene umfasst, die geeignet ist, die Faserstoffbahn in einer Position zu stabilisieren.

Die Faserstoffbahn wird dabei im Abstand zur Kamera stabilisiert, um einen gleichmäßigen Fokusbereich zu ermöglichen. Die Führungsschiene kann die Position der Faserstoffbahn durch geeignete Luftführung stabilisieren. Luftdüsen oder eine Möglichkeit Luft durch einen Unterdruck zu entfernen, kann die stabilisierende Wirkung ebenfalls unterstützen. Luftdüsen können auch direkt in die Führungsschiene integriert werden.

Im Folgenden wird die Erfindung anhand schematischer, nicht maßstäblicher Figuren weiter erläutert.
Figur 1 zeigt schematisch einen Schnitt durch eine Vorrichtung zur Ermittlung der Faserorientierung gemäß einem Aspekt der Erfindung
Figur 2 mit den Teilfiguren 2a, 2b, 2c zeigt eine mögliche Ausführungsform für Beleuchtungsmittel zum Einsatz in einer Vorrichtung gemäß einem Aspekt der Erfindung,

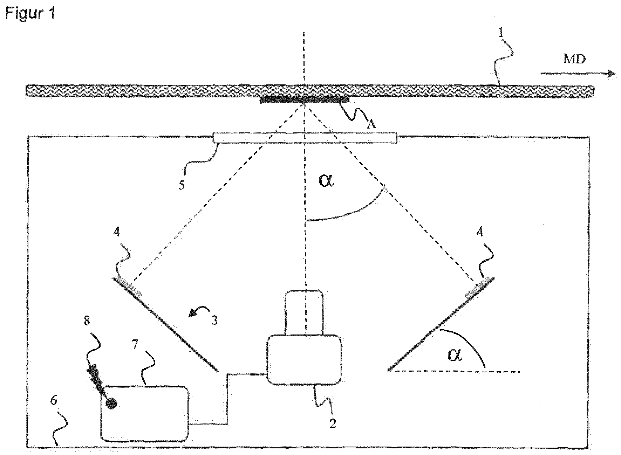

Figur 1 zeigt eine Faserstoffbahn 1, beispielsweise eine Papierbahn 1 oder eine Verpackungsbahn 1, die in Maschinenrichtung MD bewegt ist. Die Geschwindigkeit der Faserstoffbahn 1 kann dabei mehr als 1000 m/min, insbesondere mehr als 1200 m/min betragen. Es ist dabei eine Kamera 2 vorgesehen, um ein digitales Bild einer Fläche A von einer Oberfläche der Faserstoffbahn 1 aufzunehmen. Bei der in Figur 1 dargestellten Ausführung wird dabei ein Bild der Unterseite aufgenommen. Alternativ oder zusätzlich kann eine Kamera 2 auch oberhalb der Faserstoffbahn 1 angeordnet sein, um ein Bild der Oberseite aufzunehmen. Weiterhin sind Beleuchtungsmittel 3 vorgesehen, um die Oberfläche der Faserstoffbahn 1 zu beleuchten. Dabei umfasst die beleuchtete Fläche die Fläche A, welche mittels der Kamera 2 erfasst wird. Vorteilhafterweise umfasst die Fläche A ein Quadrat von 5mmx5mm, insbesondere von 10mmx10mm. Die aufgenommene Fläche muss dabei kein Quadrat sein. In Tests wurden z.B. erfolgreich Aufnahmen von 11mm x 9 mm erstellt.

Die Beleuchtungsmittel 3 umfassen eine Vielzahl von Lichtquellen 4. Diese Lichtquellen 4 sind in Figur 1 als LEDs 4 ausgeführt, welche gleichmäßig und ringförmig um die Kamera 2 herum angeordnet sind. Wie beschrieben ist eine ungerade Anzahl von Lichtquellen 4 vorteilhaft.

Die Beleuchtungsmittel 3 sind dabei so ausgeführt, dass das Licht der Lichtquellen 4 in einem Winkel α - gemessen von der Senkrechten - auf die Oberfläche der Papierbahn 1 trifft. Dieser Winkel α ist vorteilhafterweise < 45°, das heißt, dass das Licht vergleichsweise steil auf die Oberfläche trifft. Dadurch wird beispielsweise verhindert, dass ein Schattenwurf der Oberflächentopographie das Messergebnis verfälscht.

Die Beleuchtungsmittel 3 sind zudem dazu eingerichtet, die Fläche A, von der ein digitales Bild aufgenommen wird für einen Zeitraum Δt von maximal 1 [µs], insbesondere von 500 [ns] oder weniger zu beleuchten. Mit der aktuellen Technologie sind sogar deutlich kürzere Beleuchtungszeiten von 200 [ns] oder weniger mit vertretbarem Aufwand realisierbar.

In der Ausführung der Figur 1 weist die Vorrichtung eine Einhausung 6 beispielsweise eines Kastens auf Metall auf. Diese Einhausung 6 bildet die Außenwand der Sensoreinheit. In der Einhausung 6 ist eine Platte 5 vorgesehen, die z.B. aus Glas oder aus Kunststoff bestehen kann, und die für das Licht der Lichtquellen 4 ganz oder Großteils transparent ist. Durch diese Platte 5 wird sowohl die Faserstoffbahn 1 beleuchtet, als auch das digitale Bild aufgenommen. Um Verschmutzungen, die sich auf der Platte 5 ablagern können zu entfernen, können zudem auch noch Reinigungsvorrichtungen vorgesehen sein, z.B. Düsen zur Spülung der Platte 5 mit Luft oder Wasser. Diese Reinigungsvorrichtungen sind in der Figur 1 nicht explizit dargestellt.

Inder Ausführung der Figur 1 ist die Recheneinheit 7 auf innerhalb der Sensoreinheit, also im Inneren der Einhausung 6 angeordnet. Weiterhin ist eine Übertragungseinrichtung 8 vorgesehen, um Daten von der Recheneinheit 7 zu einer Empfangsstation zu übermitteln, die Außerhalb der eigentlichen Papiermaschine angeordnet ist. Da in dieser Ausführung die Berechnung der Faserorientierung durch die Recheneinheit 7 im Inneren der Sensoreinheit erfolgt, muss die Übertragungseinrichtung 8 lediglich sehr geringe Datenmengen übertragen.

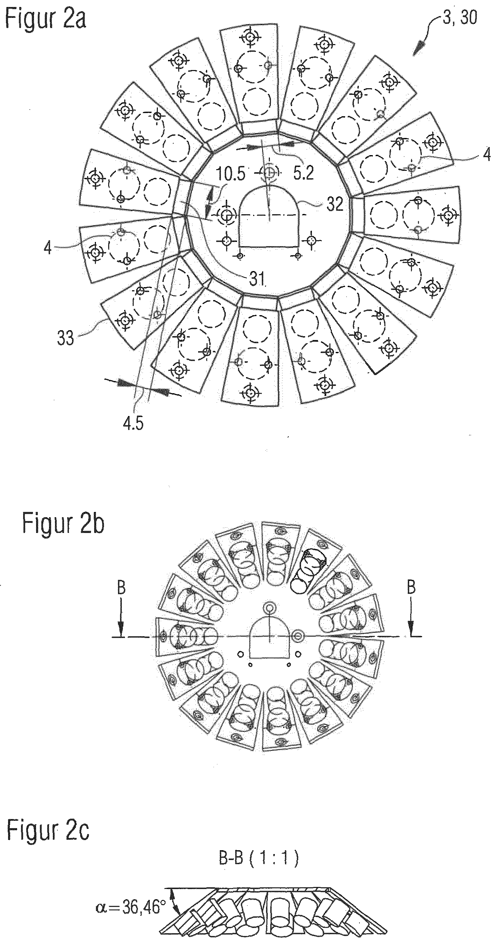

Figur 2, bzw. die Teilfiguren 2a, 2b und 2c zeigen Details von Beleuchtungsmitteln 3, die insbesondere auch in der in Figur 1 gezeigten Ausführung verwendet werden können. Beleuchtungsmittel 3 dieser Art stellen im Übrigen auch für sich alleine eine erfinderische Idee dar und können unabhängig von der Anwendung zur Bestimmung der Faserorientierung in anderen Bereichen eingesetzt werden.

Ein zentrales Element dieser Beleuchtungsmittel 3 stellt eine Leiterplatte 30 dar. In der Draufsicht der Figur 2a erkennt man, dass diese Leiterplatte 30 in Form eine Blume ausgeführt ist. Im Zentrum der Leiterplatte 30 ist eine Aussparung 32 für die Kamera 2 vorgesehen. Um das Zentrum herum sind symmetrisch 15 "Blütenblätter" 33 angeordnet. Bei diesen Blütenblättern 33 handelt es sich um Teile der Leiterplatte 30, die jeweils über eine flexible Verbindung 31 mit dem Zentrum verbunden sind. Diese Blütenblätter 33 sind vorteilhafterweise alle identisch ausgeführt. Sie umfassen jeweils eine Lichtquelle 14. Als Lichtquelle geeignet sind beispielsweise "Oslon SSL 80" LEDs der Firma Osram.

Über die flexible Verbindung können die Blütenblätter 33 so eingestellt werden, dass das Licht der Lichtquellen 14 unter dem gewünschten Winkel α auf die Faserstoffbahn 1 auftrifft. Figur 2 b zeigt perspektivisch die Leiterplatte 30 der Figur 2a, bei der die Blütenblätter 33 bereits an der flexiblen Verbindung 31 geneigt wurden. Wie beschrieben sollte der Winkel kleiner als 45° sein. Figur 2c zeigt einen Schnitt durch Figur 2b entlang der Linie B. In der in Figur 2 gezeigten Ausführung wurden die Blütenblätter um einen Winkel a zwischen 36° und 37° eingestellt.

### Bezugszeichenliste

- 1: Faserstoffbahn
- 2: Kamera
- 3: Beleuchtungsmittel
- 4: Lichtquelle
- 5: Platte
- 6: Einhausung
- 7: Recheneinheit
- 8: Übertragungseinrichtung
- 30: Leiterplatte
- 31: Flexible Verbindung
- 32: Aussparung
- 33: "Blütenblatt"
- A: Bildfläche
- MD: Maschinenrichtung

## Patentansprüche

1. Verfahren zur Ermittlung der Faserorientierung einer bewegten Faserstoffbahn (1), insbesondere einer Papier-, Karton- oder Zellstoffbahn (1), umfassend die Schritte
- Beleuchten einer Fläche A der Faserstoffbahn (1) für einen Zeitraum Δt
- Aufnahme eines digitalen Bildes der beleuchteten Fläche A
**dadurch gekennzeichnet, dass**
der Zeitraum Δt maximal 1 [µs], insbesondere maximal 500 [ns] beträgt, und das digitale Bild eine so hohe Auflösung aufweist, dass die Kantenlänge eines Pixels weniger als 20 [µm], insbesondere weniger als 10 [µm] entspricht.

2. Verfahren gemäß Anspruch 1, **dadurch gekennzeichnet, dass** das Verfahren die Berechnung einer Faserorientierung aus den Daten des digitalen Bildes umfasst.

3. Verfahren gemäß einem der vorherigen Ansprüche, **dadurch gekennzeichnet, dass** die Beleuchtung der Fläche A durch Licht mit Wellenlängen im Bereich von 450 nm - 780 nm, insbesondere zwischen 450 nm und 550 nm oder im Bereich 600 nm - 700 nm erfolgt.

4. Verfahren gemäß einem der vorherigen Ansprüche, **dadurch gekennzeichnet, dass** die zur Beleuchtung verwendeten Wellenlängen eine Bandbreite von maximal 100 nm, insbesondere maximal 50 nm aufweisen.

5. Verfahren gemäß einem der vorherigen Ansprüche, **dadurch gekennzeichnet, dass** das Licht der Beleuchtung unter einem Winkel α auf die Faserstoffbahn (1) trifft, welcher maximal 45°, insbesondere zwischen 25° und 40° von der Senkrechten abweicht.

6. Verfahren gemäß einem der vorherigen Ansprüche, **dadurch gekennzeichnet, dass** die beleuchtete Fläche A ein Quadrat von zumindest 5 mm x 5 mm, insbesondere von 10 mm x 10 mm umfasst.

7. Verfahren gemäß einem der vorherigen Ansprüche, **dadurch gekennzeichnet, dass** sich die Faserstoffbahn (1) mit einer Geschwindigkeit von mehr als 1000 m/min, insbesondere mehr als 1200 m/min bewegt.

8. Vorrichtung zur Ermittlung der Faserorientierung eine bewegten Faserstoffbahn (1), insbesondere einer Papier-, Karton- oder Zellstoffbahn (1), umfassend Beleuchtungsmittel (3) zum Beleuchten einer Fläche A der Faserstoffbahn, sowie eine Kamera (2) zur Aufnahme eines digitalen Bildes der beleuchteten Fläche A der Faserstoffbahn (1), **dadurch gekennzeichnet, dass** die Beleuchtungsmittel (3) dazu eingerichtet sind, die Faserstoffbahn (1) für einen Zeitraum von [1µs] oder weniger zu beleuchten, sowie die Kamera (2) derart eingerichtet ist, dass die Kantenlänge eines Pixels maximal 20 [µm], insbesondere maximal 10 [µm] entspricht.

9. Vorrichtung gemäß Anspruch 8, **dadurch gekennzeichnet, dass** die Vorrichtung zudem eine Recheneinheit (7) umfasst, die dazu eingerichtet ist, aus den Daten eines digitalen Bildes der Kamera (2) eine Faserorientierung zu berechnen.

10. Vorrichtung gemäß einem der Ansprüche 8 oder 9, **dadurch gekennzeichnet, dass** die Beleuchtungsmittel (3) mehrere Lichtquellen (4), insbesondere mehrere LEDs (4) umfassen, welche gleichmäßig und ringförmig um die Kamera (2) herum angeordnet sind.

11. Vorrichtung gemäß einem der Ansprüche 8 bis 10, **dadurch gekennzeichnet, dass** die Beleuchtungsmittel (3) eine ungerade Anzahl von Lichtquellen (4) aufweisen.

12. Vorrichtung gemäß einem der Ansprüche 8 bis 11, **dadurch gekennzeichnet, dass** die Beleuchtungsmittel (3) so ausgeführt sind, dass das Licht unter einem Winkel α auf die Faserstoffbahn (1) trifft, welcher maximal 45°, insbesondere zwischen 25° und 40° von der Senkrechten abweicht.

13. Vorrichtung gemäß einem der Ansprüche 8 bis 12, **dadurch gekennzeichnet, dass** zwischen den Beleuchtungsmitteln (3) und der Faserstoffbahn (3) und/oder zwischen der Kamera (2) und der Faserstoffbahn (1) eine Platte (5) angeordnet ist, die für das Licht der Beleuchtungsmittel (3) ganz oder weitgehend transparent ist.

14. Vorrichtung gemäß einem der Ansprüche 8 bis 13, **dadurch gekennzeichnet, dass** die Beleuchtungsmittel (3) zumindest kurzzeitig dazu geeignet sind, mit einer Stromstärke von mindestens 10 [A] betrieben zu werden.

15. Vorrichtung gemäß einem der Ansprüche 8 bis 14, **dadurch gekennzeichnet, dass** die optischen Eigenschaften und die Blenden-Einstellungen mit Blenden zwischen F4 und F16, der Linse der Kamera eine Auflösung ermöglicht, die **einer** Kantenlänge eines Pixels von maximal 20 [µm], insbesondere maximal 10 [µm] entspricht.

16. Vorrichtung gemäß einem der Ansprüche 8 bis 15, **dadurch gekennzeichnet, dass** die Vorrichtung eine Führungsschiene umfasst, die geeignet ist, die Faserstoffbahn (1) in einer Position zu stabilisieren.

## Claims

1. A method for determining the fibre orientation of a moving fibrous web (1), in particular a paper, board or pulp web (1), comprising the steps
- illuminating an area A of the fibrous web (1) for a period of time Δt
- capturing a digital image of the illuminated area A
**characterised in that**
the time interval Δt is at most 1 [µs], in particular at most 500 [ns], and the digital image has a resolution such that the edge length of a pixel is less than 20 [µm], in particular less than 10 [µm].

2. A method according to claim 1, **characterised in that** the method comprises calculating a fibre orientation from the data of the digital image.

3. A method according to one of the preceding claims, **characterised in that** the surface A is illuminated by light with wavelengths in the range of 450 nm - 780 nm, in particular between 450 nm and 550 nm or in the range 600 nm - 700 nm.

4. A method according to one of the preceding claims, **characterised in that** the wavelengths used for illumination have a bandwidth of at most 100 nm, in particular at most 50 nm.

5. A method according to one of the preceding claims, **characterised in that** the light used for illumination strikes the fibre web (1) at an angle α which deviates from the vertical by a maximum of 45°, in particular between 25° and 40°.

6. A method according to one of the preceding claims, **characterised in that** the illuminated area A comprises a square of at least 5 mm × 5 mm, in particular 10 mm × 10 mm.

7. A method according to one of the preceding claims, **characterised in that** the fibre web (1) moves at a speed of more than 1000 m/min, in particular more than 1200 m/min.

8. Apparatus for determining the fibre orientation of a moving fibrous web (1), in particular a paper, board or pulp web (1), comprising illumination means (3) for illuminating an area A of the fibrous web, and a camera (2) for capturing a digital image of the illuminated area A of the fibrous web (1), **characterised in that** the illumination means (3) are arranged to illuminate the fibrous web (1) for a period of [1 µs] or less, and the camera (2) is arranged such that the edge length of a pixel corresponds to a maximum of 20 [µm], in particular a maximum of 10 [µm].

9. A device according to claim 8, **characterised in that** the device further comprises a processing unit (7) configured to calculate a fibre orientation from the data of a digital image from the camera (2).

10. A device according to one of claims 8 or 9, **characterised in that** the illumination means (3) comprise a plurality of light sources (4), in particular a plurality of LEDs (4), which are arranged uniformly and in a ring-shaped pattern around the camera (2).

11. Apparatus according to any one of claims 8 to 10, **characterised in that** the illumination means (3) comprise an odd number of light sources (4).

12. Apparatus according to any one of claims 8 to 11, **characterised in that** the illumination means (3) are designed such that the light strikes the fibre web (1) at an angle α which deviates from the vertical by a maximum of 45°, in particular between 25° and 40°.

13. Apparatus according to any one of claims 8 to 12, **characterised in that** a plate (5), which is completely or largely transparent to the light from the illuminating means (3), is arranged between the illuminating means (3) and the fibre web (1) and/or between the camera (2) and the fibre web (1).

14. Apparatus according to any one of claims 8 to 13, **characterised in that** the illumination means (3) are capable, at least for a short period, of being operated at a current of at least 10 [A].

15. Apparatus according to any one of claims 8 to 14, **characterised in that** the optical properties and the aperture settings, with apertures between F4 and F16, of the camera lens enable a resolution corresponding to a pixel edge length of at most 20 [µm], in particular at most 10 [µm], of the illuminated area A.

16. A device according to any one of claims 8 to 15, **characterised in that** the device comprises a guide rail suitable for stabilising the fibre web (1) in a position.

## Revendications

1. Procédé pour déterminer l'orientation des fibres d'une bande de matière fibreuse (1) en mouvement, en particulier d'une bande de papier, de carton ou de pâte à papier (1), comprenant les étapes suivantes
- d'éclairer une surface A de la bande de matière fibreuse (1) pendant un intervalle de temps Δt
- l'acquisition d'une image numérique de la surface A éclairée
**caractérisé en ce que**
la durée Δt est au maximum de 1 [µs], en particulier au maximum de 500 [ns], et l'image numérique présente une résolution suffisamment élevée pour que la longueur d'arête d'un pixel soit inférieure à 20 [µm], en particulier inférieure à 10 [µm].

2. Procédé selon la revendication 1, **caractérisé en ce que** le procédé comprend le calcul d'une orientation des fibres à partir des données de l'image numérique.

3. Procédé selon l'une des revendications précédentes, **caractérisé en ce que** l'éclairage de la surface A s'effectue à l'aide d'une lumière dont les longueurs d'onde sont comprises dans la plage de 450 nm à 780 nm, en particulier entre 450 nm et 550 nm ou dans la plage de 600 nm à 700 nm.

4. Procédé selon l'une des revendications précédentes, **caractérisé en ce que** les longueurs d'onde utilisées pour l'éclairage présentent une largeur de bande maximale de 100 nm, en particulier de 50 nm.

5. Procédé selon l'une des revendications précédentes, **caractérisé en ce que** la lumière d'éclairage frappe la bande de pâte à papier (1) sous un angle α qui s'écarte de la perpendiculaire de 45° au maximum, en particulier entre 25° et 40°.

6. Procédé selon l'une des revendications précédentes, **caractérisé en ce que** la surface éclairée A comprend un carré d'au moins 5 mm × 5 mm, en particulier de 10 mm × 10 mm.

7. Procédé selon l'une des revendications précédentes, **caractérisé en ce que** la bande de matière fibreuse (1) se déplace à une vitesse supérieure à 1 000 m/min, en particulier supérieure à 1 200 m/min.

8. Dispositif destiné à déterminer l'orientation des fibres d'une bande de matière fibreuse (1) en mouvement, en particulier d'une bande de papier, de carton ou de pâte à papier (1), comprenant des moyens d'éclairage (3) destinés à éclairer une surface A de la bande de matière fibreuse, ainsi qu'une caméra (2) destinée à enregistrer une image numérique de la surface éclairée A de la bande de matière fibreuse (1), **caractérisé en ce que** les moyens d'éclairage (3) sont agencés pour éclairer la bande de matière fibreuse (1) pendant une durée de [1 µs] ou moins, et **en ce que** la caméra (2) est agencée de telle sorte que la longueur d'arête d'un pixel corresponde à 20 [µm] au maximum, en particulier à 10 [µm] au maximum.

9. Dispositif selon la revendication 8, **caractérisé en ce que** le dispositif comprend en outre une unité de calcul (7) agencée pour calculer une orientation des fibres à partir des données d'une image numérique provenant de la caméra (2).

10. Dispositif selon l'une des revendications 8 ou 9, **caractérisé en ce que** les moyens d'éclairage (3) comprennent plusieurs sources lumineuses (4), en particulier plusieurs LED (4), qui sont disposées de manière uniforme et en anneau autour de la caméra (2).

11. Dispositif selon l'une des revendications 8 à 10, **caractérisé en ce que** les moyens d'éclairage (3) comportent un nombre impair de sources lumineuses (4).

12. Dispositif selon l'une des revendications 8 à 11, **caractérisé en ce que** les moyens d'éclairage (3) sont conçus de telle sorte que la lumière frappe la bande de pâte (1) sous un angle α qui s'écarte de la perpendiculaire de 45° au maximum, en particulier entre 25° et 40°.

13. Dispositif selon l'une des revendications 8 à 12, **caractérisé en ce qu'**une plaque (5), qui est entièrement ou largement transparente à la lumière des moyens d'éclairage (3), est disposée entre les moyens d'éclairage (3) et la bande de pâte à papier (1) et/ou entre la caméra (2) et la bande de pâte à papier (1).

14. Dispositif selon l'une des revendications 8 à 13, **caractérisé en ce que** les moyens d'éclairage (3) sont aptes, au moins pendant un court instant, à fonctionner avec une intensité de courant d'au moins 10 [A].

15. Dispositif selon l'une des revendications 8 à 14, **caractérisé en ce que** les propriétés optiques et les réglages d'ouverture compris entre F4 et F16 de l'objectif de la caméra permettent d'obtenir une résolution correspondant à une longueur de bord d'un pixel de 20 [µm] au maximum, en particulier de 10 [µm] au maximum, de la surface éclairée A.

16. Dispositif selon l'une des revendications 8 à 15, **caractérisé en ce que** le dispositif comprend un rail de guidage apte à stabiliser la bande de matière fibreuse (1) dans une position.
